# EUROPEAN PATENT SPECIFICATION

(11) **EP 0 698 096 B1**
(45) Date of publication and mention of the grant of the patent: **26.03.1997**
(21) Application number: 94917613.5
(22) Date of filing: 10.05.1994
(51) Int. Cl.: C12N 15/12, C12N 5/10, G01N 33/50

(54) **EXPRESSION OF NTRK1-DERIVED ONCOGENES IN PC12 PHEOCHROMOCYTOMA CELLS AND USE THEREOF FOR THE SCREENING OF ANTI-TYROSINE KINASE COMPOUNDS**
EXPRESSION VON NTRKI-ABGELEITETEN ONCOGENEN IN PCIZ PHEOCHROMOCYTOMAZELLEN UND IHRE VERWENDUNG IM AUFSPÜREN VON ANTI-TYROSINKINASE-VERBINDUNGEN
EXPRESSION D'ONCOGENES DERIVES DE NTRK1 DANS DES CELLULES DE PHEOCHRONOCYTOME PC12 ET LEUR UTILISATION POUR LE CRIBLAGE DE COMPOSES ANTI-TYROSINE-KINASE

(30) Priority: 14.05.1993 IT MI930985
(43) Date of publication of application: 28.02.1996
(73) Proprietor: ISTITUTO NAZIONALE PER LO STUDIO E LA CURA DEI TUMORI, I-20133 Milano (IT)
(72) Inventor: GRECO, Angela, I-20133 Milano (IT); PIEROTTI, Marco, A., I-20133 Milano (IT)
(74) Representative: Minoja, Fabrizio
(86) International application number: EP9401509
(87) International publication number: WO9426889

(56) References cited:
- ONCOGENE, vol.7, no.2, February 1992, pages 371-381; PETER TAPLEY ET AL: 'K252a is a selective inhibitor of the tyrosine protein kinase activity of the trk family of oncogenes and neurotrophin receptors'
- NATURE, vol.318, 7 November 1985, LONDON GB, pages 73-75; MAKOTO NODA ET AL: 'Sarcoma virus carrying ras oncogenes induce differentiation-associated properties in aneuronal cell line'
- CELL GROWTH & DIFFERENTIATION, vol.4, no.7, July 1993, pages 539-546; ANGELA GRECO ET AL: 'Expression of TRK-T1 oncogene induces differentiation of PC12 cells'

## Description

### Field of the Invention

This invention relates to the field of target-derived neurotrophic factors that regulate growth and survival of cells of the nervous system in the developing and adult vertebrate organism. In particular, this invention focuses on the mechanism of NGF-induced neuronal differentiation. The invention provides neuronal cells comprising oncogenic versions of the NGF receptor gene *NTRK*1 and displaying neuronal differentiation in the absence of NGF, eukaryotic expression vectors containing the *NTRK*1-derived oncogene *TRK*-T1*,* and a method to obtain said neuronal cells by introduction of *NTRK*1-derived oncogenes into neuronal host cells. The *in vitro* cell culture system of this invention is useful for research efforts aimed at the treatment of traumatic and neurodegenerative deseases of the central and peripheral nervous system, as well as in the screening of tyrosine-kinase inhibitors.

### Background of the Invention

Morphogenesis of the nervous system in the vertebrate organism is dependent on a complex interplay of different biochemical and molecular events, that are necessary to insure that, during growth and migration, neural cells will find, and project to, the adequate target regions in the developing tissue. Besides intercellular interactions and interactions of cells with signal-providing components of the extracellular matrix, target-derived neurotrophic factors that regulate cellular growth and survival play an essential role during these processes. Also, maintenance and viability of neuronal connections in the adult organism is believed to be strongly influenced by the availability of, and responsiveness to, neurotrophic factors. The discovery and isolation of nerve growth factor (NGF) that has been shown to control development, differentiation and survival of neurons of the sympathetic nervous system and parts of the sensory and central nervous system (Levi-Montalcini, R. and Angeletti, P. (1968). *Physiol. Rev.* 48, 534-569; Thoenen, H., and Barde, Y. A. (1980). *Physiol. Rev.* 60, 1284-1325; Whittemore, S. R., and Seiger, A. (1987). *Brain Res.* 434, 439-464; Thoenen, H., et al. (1987). *Rev. Physiol. Biochem. Pharmacol.* 109, 145-178), has provided the first example of a trophic factor active in neural tissue. Meanwhile, additional neurotrophic factors that share structural and functional similarities with NGF, have been added to the growing family of the so-called "neurotrophins", underscoring the relevance of the concept of neurotrophic factors *in vivo.*

The possible relationship of NGF to several neurodegenerative diseases of the central and peripheral nervous system makes the investigation of NGF induced signal transduction a subject of main scientific interest. The cholinergic neurons of the basal forebrain nuclei, for instance, known to be important for cognitive functions, consistently undergo degenerative changes in cases of Alzheimer's desease. These neurons are responsive to NGF (Hefti, F., and Weiner, W. J. (1986). *Ann. Neurol.* 20, 275-281), and their degeneration could be associated with a decrease in NGF responsiveness or limitations in the availability of NGF. Also, the poor capacity of mammalian central nervous system neurons to regenerate upon injury might be in part due to an insufficient supply of target-derived NGF or other neurotrophins and could perhaps be improved by the exogenous application of these factors. A detailed knowledge of the molecular events triggered by NGF in neuronal cells will be important for this field and promises to provide ways for the development of new concepts for the treatment of diseases of the nervous system.

These perspectives have prompted the search for NGF receptors on neuronal cell surfaces, with the final goal to uncover the molecular mechanisms underlying NGF action. NGF was found to interact with two types of receptors on neuronal cell surfaces which can be discriminated biochemically by their different affinity as the low and the high affinity NGF receptor. The molecular basis of the low affinity NGF receptor (LNGFR) is represented by a transmembrane glycoprotein with a molecular weight of 75 000 daltons that binds NGF with a K_{d} of 10⁻⁹ M (p75 , also designated p75^{NGFR}; Johnson, D., et al. (1986). *Cell* 47, 545-554; Radeke, M. J., et al. (1987). *Nature* 325, 593-597) and is expressed on neuronal as well as on nonneuronal cells. However, the low degree of cell type specificity of its expression and the lack of significant homologies with sequences in the intracellular domains of other transmembrane receptors known to mediate signal transduction, make it seem unlikely that p75 can serve as a functional receptor by itself. Furthermore, a transmembrane glycoprotein of 140 000 daltons (NTRK1; also designated Trk, TrkA, p140^{trk}, gp140^{trk} or p140^{prototrk} in the literature), a member of the tyrosine-kinase family of receptors which is encoded by the *NTRK*1 protooncogene and had initially been identified in its rearranged oncogenic version as the transforming principle underlying certain tumors in nonneural tissues (Martin-Zanca, D., et al. (1986). *Nature* 319, 743-748), was found to be a receptor for NGF (Kaplan, D., et al. (1991). *Science* 252, 554-558; Klein, R.,et al. (1991). *Cell* 65, 189-197), and has been proposed to account for, or be part of, the high affinity NGF receptor (HNGFR). While there is up to now still considerable debate, whether NTRK1 is the only component of the HNGFR *in vivo ,* or has to act in concert with p75^{NGFR} or other accessory proteins, it has been shown that the NTRK1 protein alone is sufficient for binding of NGF (although the equilibrium binding constant of this interaction is a matter of controversy; see Klein, R., et al. (1991). *Cell* 65, 189-197; Kaplan, D., et al. (1991). *Science* 252, 554-558; Hempstead, B., et al. (1991). *Nature* 350, 678-683) and for mediating NGF-induced survival and differentiation of neuronal cells (Ibáñez, C. F., et al. (1992). *Cell* 69, 329-341).

The identification and molecular cloning of functional NGF receptors has opened the possibility to systematically monitor the biochemical events occurring in the signal transduction cascade of the cell that lead to the metabolical and morphological changes observed upon NGF application, starting with the formation of the receptor-ligand complex and ending with long term changes in gene expression and cellular metabolism. The most commonly used system to characterize biochemically these events are the rat PC12 pheochromocytoma cells, derived from an adrenal tumor of adrenergic neural crest cell origin (Tischler, A. S., and Greene, L. A. (1975). *Nature* 258, 341-342) that express both NGF receptor subtypes, the LNGFR and the HNGFR. As a result of NGF treatment for several days, PC12 cells stop dividing, develop excitable membranes, grow neuronal processes and differentiate into a sympathetic neuronal phenotype. These features go along with straightforward and well described culture conditions, having made PC12 cells the paradigm for an *in vitro* cell culture system to study the molecular events required for the induction of neuronal differentiation. Despite the advantages of this system, however, the rapidly accumulating knowledge about NGF-induced changes in the cellular metabolism has lead into a dilemma which can be best exemplified by a comparison of the effects evoked by NGF and epidermal growth factor (EGF) in PC12 cells.

Similarly to NGF, EGF exerts its biological effects on PC12 cells by the interaction with a transmembrane tyrosine kinase receptor, encoded by the *erbB* protooncogene. In contrast to NGF which induces withdrawal from the cell cycle and differentiation, EGF acts as a mitogen and promotes proliferation. The second messenger mechanisms required to mediate such divergent biological effects were expected to be considerably different. Surprisingly, however, both factors were found to initiate intracellular signal transduction events and changes in gene expression which are nearly indistinguishable from each other. For example, with respect to its effects on gene expression, both NGF and EGF treatment of PC12 cells leads to the activation of immediate early genes to a comparable level and with a similar time course (Greenberg, M. E., et al. (1985). *Biol. Chem.* 260, 14101-14110; Bartel, D. P., et al. M. E. (1989). *Genes Dev.* 3, 304-313). Also, almost identical increases in cellular reactions like membrane ruffling (Seeley, P. J., et al. L. A. (1984). *J. Cell Biol.* 98, 417-426), Na⁺/K⁺ transport (Boonstra, J., et al. (1983). *J. Cell Biol.* 97, 92-98), cell adhesion, and glucose uptake (Huff,K., et al. (1981). *J. Cell Biol.* 88, 189-198) have been observed using either NGF or EGF. Moreover, the pattern of increased tyrosine phosphorylation of cellular proteins is very much alike for EGF- and NGF treated cells (Maher, P. (1988). *Proc. Natl. Acad. Sci. USA* 85, 6788-6791). Interestingly, NGF, like EGF, triggers phosphorylation of phospholipase C-τ (Kim, U.-H., et al. (1991). *J. Biol. Chem.* 266, 1359-1362; Vetter, M. L., et al. (1991). *Proc. Natl. Acad. Sci. USA* 88, 5650-5654), which was initially thought to be a signalling event characteristic for the transduction of mitogenic signals.

In the course of studies addressing the role of oncogenes in the establishment of the neuronal phenotype, it was shown by introduction of either activated oncogenes or oncoproteins into PC12 cells that the proteins encoded by Ha-*ras ,* N-*ras* and retroviral *ras* -derived genes are able to induce PC12 cell differentiation (Guerrero, I., et al. (1986). *J. Cell. Physiol.* 129, 71-76; Bar-Sagi, D., and Feramisco, J. R. (1985). *Cell* 42, 841-848; Noda, M., et al. Y. (1985). *Nature* 318, 73-75). Also, a promoting role of p21^{ras} for survival and neurite outgrowth of freshly dissociated chicken embryonic neurons could be demonstrated (Borasio, G. D., et al. (1989). *Neuron* 2, 1087-1096), suggesting an involvement of ras protein in the signalling pathway used by NGF. Further evidence for this notion was provided by the observation that, upon microinjection, anti-p21^{ras} antibodies can block NGF-induced differentiation in PC12 cells (Hagag, N., et al. (1986). *Nature* 319, 680-682.). Transfection experiments of ras dominant negative mutants in PC12 cells indicated that, on the molecular level, p21^{ras} affects events downstream of the NGF-induced autophosphorylation of the NTRK1 protein, (Thomas, S. M., et al. (1992). *Cell* 68, 1031-1040; Wood, K. W., et al. (1992). *Cell* 68, 1041-1050). However, phosphorylation of MAP kinases and RSK is also known to be induced by EGF (Mutoh, T., et al. (1988). *J. Biol. Chem.* 263, 15853-15856; Miyasaka, T., et al. (1990). *J. Biol. Chem.* 265, 4730-4735.) and other growth factors. Moreover, activation of cellular ras protein by EGF, as judged by GTP binding and hydrolysis, is indistinguishable from the activation by NGF (Qui, M. S., and Green, S. H. (1991). *Neuron* 7, 937-946.), indicating that p21^{ras} activity and events downstream of p21^{ras} activation are affected in a comparable manner upon treatment with either NGF or EGF.

Similarly to *ras* oncogene products, the protein encoded by the *v-src* oncogene was found to be an inducer of neuronal differentiation (Alema, S., et al. (1985). *Nature* 316, 557-559.) and it has been suggested that its function is required prior to ras activation (Kremer, N. E., et al. (1991). *Neuron* 7, 937-946.). The function of the *src* protooncogene product, pp60^{c-src}, is unknown. It has been shown, however, that it is probably also involved in the transduction of signals triggered by the cell adhesion molecules N-CAM, L1 and MAG (Atashi, J. R., et al. (1992). *Neuron* 8, 831-842.). The molecular basis of a signalling pathway exclusively used by NGF, if it should exist at all, therefore remains unclear.

Considering the particular biological effects of NGF on the one side and the absence of any obvious differences to other growth factors in the recruitment of intracellular signalling molecules on the other side, it is reasonable to focus the attention on the first steps of NGF-induced signal transduction. In addition to the above mentioned second messenger systems, activation of signals that have not yet been focused on might be initiated here, possibly by the involvement of accessory proteins acting in concert with NTRK1. This idea is supported by the observation that, in contrast to its effects on neuronal cells, NGF is a powerful mitogen for NIH3T3 fibroblast-like cells that have been manipulated to express NTRK1 (Cordon-Cardo, C., et al. (1991). Cell 66, 173-183.). Also, recent studies have shown a direct interaction of the NTRK1 protein with the serine/threonine kinase ERK1 in PC12 cells (Loeb, D. M., et al. (1992). *Neuron* 9*,* 1053-1065.), which possibly results in the subsequent phosphorylation of other intracellular proteins.

In order to gain more information about the molecular mechanisms of NGF action it is therefore important to investigate the initial decisive steps of NGF-mediated signalling. In this respect, it would be desirable to have an *in vitro* systems providing novel approaches to study NTRK1 activation and the subsequent early signalling steps on the molecular level. In the last years the relationship between cancer and the biochemical pathway of the signal transduction has become more and more evident.

It is well-established that the perturbation of said pathways in one of their different passages induces tumoral transformation in this respect, a particular aspectis represented by the deregulation of the genes having tyrosine-kinase activity, mainly representing receptors for growth factors or other factors. This deregulation, which transforms the genes in oncogenes, may be due to different causes, but it usually always exhibits a non -programed a tyrosine-kinase activity which transmits the cells continuos proliferative signals causing tumoral transofrmations it is therefore evident the extreme importance of substances able to interrupt this mechanism by dodulating or eliminating the tyrosine-kinase activity of the above disclosed oncogenes. These substances could also prevent the tumoral transformation.

A novel *in vitro* systems to study NGF action should be easy to handle and should offer straightforward possibilites to monitor cellular responses to any experimental manipulations.

### Surmmary of the Invention:

The present invention addresses the problems raised in the prior art by providing an *in vitro* model system for neuronal differentiation that allows to study the basic mechanisms of NGF-specific signal transduction at the level of the NGF receptor and it may also be used to screen compounds having inhibiting action on tyrosine-kinase activity of the protein encoded by TRK-T1 and of the proteins encoded by other oncogenes sharing with TRK-T1 the catalytic site having tyrosine-kinase activity. The system of the invention is provided by neuronal cells, characterized in that they comprise a gene coding for an oncogenic derivative of the NGF-receptor NTRK1, said gene causing neuronal differentiation of said cells in the absence of NGF. Although these *NTRK1*-derived oncogenes have transforming activity in certain tumors, it was found that their introduction into a neuronal cell surprisingly induces neuronal differentiation. This process is independent of the presence of NGF but interestingly displays the major characteristics of the differentiation process observed with normal neuronal cells upon NGF application. In contrast to normal NGF-treated neuronal cells, however, the cells transformed with the *NTRK*1-derived oncogenes undergo degenerative processes after a certain period in culture, indicating that NGF-triggered NTRK1 protein might transduce additional signals needed for viability and maintenance of neuronal cells compared to the truncated receptors. The *in vitro* system of this invention therefore allows to compare NTRK1 with its structurally altered oncogenic derivatives with respect to the molecular mechanisms underlying their signalling activity and the recruitment of accessory molecules. Since this invention can be applied to neuronal cell lines, preferably to PC12 cells, establishment of the system is straightforward and the characteristics of the differentiation process can be easily monitored. In accord with the present invention, the eukaryotic expression vectors pC24A and pC24B, containing the *NTRK*1-derived oncogene *TRK*-T1 in sense and antisense orientation, respectively, are provided, and a method of obtaining neuronal cells displaying NGF-independent neuronal differentiation, characterized by transfection of vectors containing a gene coding for an oncogenic derivative of the NGF-receptor NTRK1 into neuronal host cells. The system of the invention, providing an useful screening method in the pharmacological search of compound having tyrosine-kinase activity, may be generalized and adapted to other tyrosine-kinase in oncogenic form.

### Brief Description of the Drawings:

Fig. 1 shows a schematic representation of vector pC24A used for transfection of PC12 cells. The vector contains the cDNA sequence of the *TRK*-T1 oncogene, indicated by the shaded and hatched boxes designated TPR and NTRK1, respectively, in sense orientation relative to the Cytomegalovirus promotor (CMV) and the SV40 polyadenylation signal (SV40 pA). Bold lines represent polylinker sequences. The positions of cleavage sites for BamHI (B), EcoRI (E) and HindIII (H) are indicated.

Fig. 2 shows the morphology of PC12 cells 4 days after transfection with pC24A (A) and pC24B (C), after treatment with NGF (50 ng/ml) for 3 days (B), and under normal culture conditions (D).

Fig. 3 shows detection of the *TRK*-T1 gene in transfected PC12 cells by PCR amplification using *TRK*-T1-specific primers followed by analysis of the amplification products on a 4% Nusieve agarose gel (3:1). Amplification was performed on low molecular weight DNA extracted from PC12 cells transfected with pC24A (lane 1), pC24A (lane 2), pLM6 (lane 3), and from mock transfectants (lane 4). The size of the marker bands (lane 5) is given on the right hand side. The size of the amplified fragments is indicated on the left hand side.

Fig. 4 shows detection of TRK-T1 and TRK oncoproteins in transfected PC12 cells by Western blotting and detection with an antiserum specific for the carboxy-terminal domain of NTRK1. Cells were transfected with pC24A (lane 1) or pDM16 (lane 2) and harvested after 5 days in culture. The position of bands representing the TRK-T1 (p55) and TRK (p70) proteins are indicated on the left hand side. The molecular weight of marker bands, run on the gel in parallel, is indicated on the right hand side.

Fig. 5 shows degeneration of PC12 cells, transfected with vector pC24A and grown in the presence of geneticin (400 µg/ml), after 10 days in culture. Photographs were taken from two independent experiments (A and B).

Fig. 6 shows the inhibitory effect of K252a on NGF- and TRK-T1-mediated neurite outgrowth of PC12 cells. Cells grown in the presence of NGF (50 ng/ml; A, B) or cells transfected with pC24A (C, D) were cultured in the presence (A, C) or absence (B, D) of K252a (200 nM) for 4 days.

Fig. 7 shows the detection of the VGF8 gene product on PC12 cells by immunoflourescence staining. *TRK*-T1-transfected cells fixed after three days in culture (A, B) and cells treated with NGF (50 ng/ml) for 1 day (C, D) were stained with preimmune serum (A, C) or aVX (B, D) antiserum.

Fig. 8 shows the effect of the D11 antibody on PC12 cell differentiation. Cells transfected with *TRK*-T1 (A) or treated with NGF (50 ng/ml; B) were treated with D11 antibodies for 3 days.

Fig. 9 shows PC12 cell differentiation induced by the *TRK* oncogene. Cells transfected with vector pDM16 were cultured in the absence (A) or presence (B) of K252a (200 nM) for 3 days.

### Detailed Description of the Invention:

The *in vitro* model system for neuronal differentiation of this invention can be set up with any neuronal cell type that is responsive to NGF. Preferably, the rat PC12 pheochromocytoma cell line is used, which can be grown under well described and straightforward culture conditions. PC12 cells are widely used and can be obtained from ATCC (Bethesda, Rockville, MD) under code number CRL 1721. However, it should be mentioned that any cell type in which NGF-induced neuronal differentiation is mediated by NTRK1, provides a molecular context that, upon transformation with oncogenic versions of *NTRK1,* leads to cellular responses similar to the effects observed with PC12 cells. Other appropriate cells would be, for example, the NGF-responsive human neuroblastoma cell lines SY5Y and LA-N-5, which express NTRK1 and display neurite outgrowth (Sonnenfeld, K. H., Ishii, D. N. (1982). *J. Neurosci. Res.* 8, 375-391.) and phosphorylation of NTRK1 upon NGF treatment (Kaplan, D. R., et al. (1991). Science 252, 554-558.).

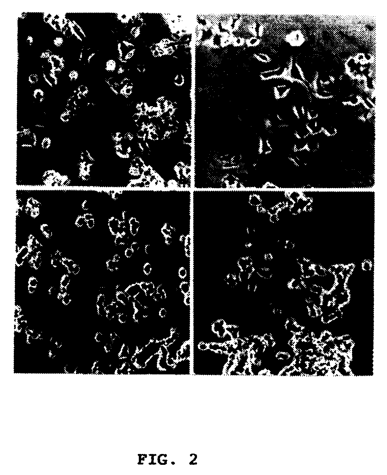

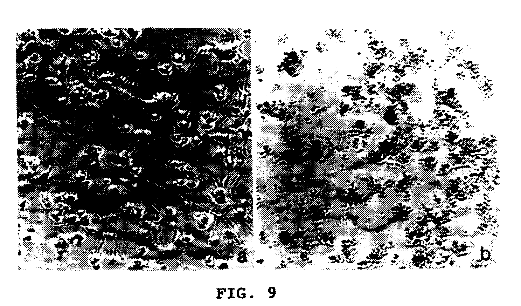

The neuronal cells are transfected with eukaryotic expression vectors containing a gene coding for an oncogenic derivative of the NGF-receptor NTRK1. A gene coding for an oncogenic derivative of NTRK1 can be any oncogene derived from the *NTRK*1 gene by pointmutation, truncation, or fusion to other genes, which includes sequences coding for the carboxy-terminal part of the NTRK1 protein and displays transforming activity in tumors or cell lines. Such oncogenes have been isolated from various tumors (Martin-Zanca, D., et al. (1986). *Nature* 319, 743-748; Bongarzone, I., et al. (1989). *Oncogene* 4, 1457-1462; Barbacid, M., Lamballe, F., Pulido, D., and Klein, R. (1991). *Biochim. Biophys. Acta 1072,* 115-127; Sozzi, G., et al. (1992). *Genes Chrom. Cancer* 5, 1-7.). The capability of these oncogenes to exert similar effects in neuronal cells is plausible, since it has been found that the gene products of the *TRK*-T1 oncogene (Greco, A., et al. (1992). Oncogene 7, 237-242.), the original *TRK* oncogene (Coulier, F., et al. (1989). *Mol. Cell Biol.* 1, 15-23) and other *NTRK1*-derived oncogenes isolated from thyroid tumors (Bongarzone, I., et al. (1989). *Oncogene* 4, 1457-1462.) are, in contrast to NTRK1, constitutively phosphorylated on tyrosine. In accord with that, transformation of PC12 cells with either the *TRK*-T1 or the *TRK* gene both was found to result in indistinguishable neuronal differentiation (see Example E and Fig. 2, 9).

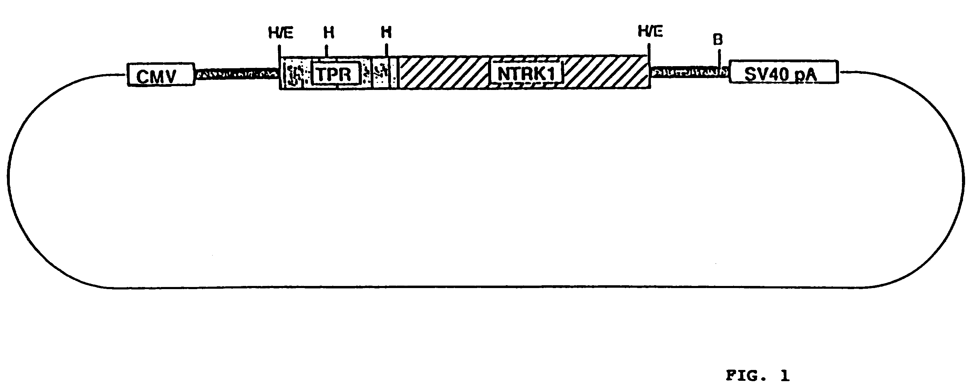

As a vector system to express *NTRK1*-derived oncogenes, eukaryotic vectors carrying the appropriate signals for expression of a foreign gene in neuronal cells are used. For this purpose, eukaryotic expression vectors designed for transient or stable integration, or vectors derived from viral systems can be employed (according to well known methods). To this purpose, episomal vectors, usually providing high expression of the inserted gene, can be used; moreover, the presence of a selectable marker allows the selection of tranofected cells. Such a vector is represented by plasmid pCEP9B (Hambor, J. E., et al. (1988). *Proc. Natl. Acad. Sci. USA* 85, 4010-4014.) which contains the Cytomegalovirus promotor (CMV) and is capable of episomal replication (Yates, J. L., et al. (1985). *Nature* 313, 812-815.). It was found that transfection of the vector pC24A, which was constructed by insertion of a *TRK*-T1 cDNA fragment into pCEP9B (see Fig. 1 and Example A), was more efficient in producing differentiating cells than the introduction of vector p24EA (Greco, A., et al. (1992). Oncogene 7, 237-242.), containing the same *TRK*-T1 cDNA insert under the control of the Adenovirus Major Late Promotor and being derived from the 91023B expression vector (Wong, G. G., et al. (1985). *Science* 228, 810-815.), designed for stable integration. Nevertheless, the effect of TRK-T1 expression on differentiation in cultures transfected with p24EA was still evident. Also, the vector pDM16 (Martin-Zanca, D., et al. (1986). *Nature* 319, 743-748) which contains the cDNA of the original *TRK* oncogene under the control of the Moloney murine sarcoma virus long terminal repeat and which is derived from the expression vector pm1SP (Blair, D. G., et al. (1981). *Science* 212, 941-943.) was found to be effective in converting the phenotype of transfected PC12 cells.

The DNA fragments coding for the *NTRK1*-derived oncogenes can be derived from genomic DNA or cDNA. These DNA fragments are inserted by conventional molecular cloning procedures known to those skilled in the art in sense and antisense orientation, with respect to the promotor, into the expression vector. Insertion of the TRK-T1 cDNA into vector pCEP9B in antisense orientation relative to the CMV promotor leads to the vector pC24B (see Example A). The antisense construct serves as a negative control, which does not not lead to a change in cellular phenotype upon transformation.

The expression vectors carrying the NTRK1-derived oncogenes are then introduced into neuronal cells. This can be achieved by several well established methods. Because of the high number of cells that can be transformed, the methods of calcium phosphate, DEAE-dextran-mediated, or liposome mediated transfection or electroporation are preferred. When PC12 cells are used, good results are obtained employing electroporation (see Examples C and D). However, the choice of an appropriate transfection method is dependent on the neuronal cell type used and can not be generalized. In case that the expression vector containing the *NTRK1*-derived oncogene does not offer the possibility for selection of transformants, a second plasmid expressing a selectable marker protein can be cotransfected. Expression of a selection marker protein allows to select for transformed cells, facilitating the analysis of the cellular differentiation process, which would otherwise be more difficult to monitor in a culture containing a high percentage of untransfected cells. A useful vector for cotransfection is pSV2neo (Southern, P. J., Berg, P. (1982). *J. Mol. Appl. Genet.* 1, 327-341.) which confers resistance to the antibiotic G418.

Subsequently to transfection, the cells are seeded into tissue culture dishes and grown in selective medium. Three days after transfection with the vectors containing the *NTRK1*-derived oncogenes in sense orientation, differentiated cells comprising long neuritic processes can be identified in the culture, which display a morphology similar to untransfected cells treated with NGF. Cultures of cells transfected with the vectors carrying the oncogenes in antisense orientation, on the other hand, show a phenotype similar to cultures of untransfected cells.

The introduction of the vector DNA can be monitored by PCR amplification of oncogene-specific fragments on extrachromosomal DNA (Hirt B., J. Mol. Biol., 26: 365-369, 1967) from cells transfected with episomal vectors and total DNA from cells transfected with integrating vectors.

Preferably, selective pressure by an antibiotic agent suited to the used selection marker, is provided throughout the entire culture period. For PC12 cells transfected with vector pC24A it was found that, during early stages of the culture (3-6 days), the differentiation of transformants is independent of the presence of G418. However, after this time, in the absence of G418 differentiated cells are no longer detectable. It is likely, that this is due to a loss of the vector DNA from the culture by dilution, which has been reported for episomal replicons in the absence of selective antibiotic pressure (Hambor, J. E., (1988). *Proc. Natl. Acad. Sci. USA* 85, 4010-4014.). This suggests that continous TRK-T1 expression is required to maintain the differentiated phenotype, which is reminiscent of the need for the continous presence of NGF to maintain NGF-induced neuronal differentiation. However, it is also possible that, in the absence of G418, the differentiated transformed cells are masked by the proliferation of the untransfected cells. Alternatively, the oncogenes of interest can be cloned into inducible expression vectors, under the control of inducible promoters such as the metallothionein promoter whose activity can be turned on by heavy metals. Following transfection, PC12 clones containing the vector can be selected; such clones can be induced to differentiate by stimulating the activity of the promoter.

The neuronal differentiation observed with the transformed cells is independent of NGF. Transformed cells differentiate in cell culture medium which has not been supplemented with NGF. Furthermore, the addition of the antibody D11, which is capable of neutralizing the activity of NGF (Cattaneo, A., et al. (1988). *J. Neurochem.* 50, 1003-1010), does not interfere with the differentiation observed in cell cultures transformed with the vectors containing the *NTRK*1-derived oncogenes. Furthermore, conditioned medium from transformed cell cultures does not induce neuronal differentiation in untransfected cells. The two latter observations exclude the possibility, that transfection of the cells induces NGF production in the transfectants, thereby leading to a change in the differentiation state by an autocrine mechanism.

Besides the morphological similarities between the neuronal differentiation observed upon transformation with NTRK1-derived oncogenes and NGF-induced cellular responses, the two processes also share similar features on the molecular and biochemical level. It was found, that the introduction of *NTRK1*-derived oncogenes into PC12 cells leads to an expressional upregulation of the protein encoded by the VGF8a gene, which is known also to be specifically induced in PC12 cells upon NGF treatment (Salton, S. R. J., et al. (1991). *Mol. Cell. Biol.* 11, 2335-2349.). This can be easily monitored by performing immunocytochemistry using the VGF8-specific antiserum aVX (Possenti, R., et al. (1989). *EMBO J.* 8, 2217-2223.) and secondary antibodies coupled to flourescent dyes. Furthermore, cultivation of the transformed cells in the presence of the inhibitor K252a inhibits the conversion of the transformants to the neuronal phenotype. K252a has the same effect on neuronal cells treated with NGF. Since K252a is a specific inhibitor of the tyrosine kinase activity of the NTRK1 protein, it can be concluded, that the TRK-T1 oncogene product mimicks to quite some extent the action of the NTRK1 protein activated by NGF.

Interestingly, after a certain time in culture, the transformed neuronal cells undergo degenerative processes. For PC12 cells it was found, that 8 days after transformation, a substantial percentage of the differentiated neuronal cells in the transformed cultures undergo degeneration, which is characterized by an increase in cell volume, the appearance of cytoplasmic vacuoles and detachment of the cells from the culture dish. This is in contrast to the behaviour of neuronal cells treated with NGF, in which the differentiated phenotype is stable as long as NGF is provided. This suggests, that in addition to its activating function of the differentiating pathway, NGF has a role in the maintenance and viability of the cells, thereby fullfilling tasks which are not covered by the activities of the NTRK1-derived oncoproteins.

The present invention can be used not only with NTRK1 derived oncogenes, but also with any other oncogene capable to induce PC12 differentiation.

The following examples further illustrate the invention.

### Example A:

### Construction of Vectors pC24A and pC24B

Vectors pC24A and pC24B are based on the eukaryotic expression vector pCEP9B (Wong, G. G., et al. (1985). *Science* 228, 810-815.). Briefly, this vector contains the Cytomegalovirus (CMV) promotor, a SV40 polyadenylation site and the neomycin resistance gene, allowing the selection of transformants after transfection with the antibiotic geneticin (G418). This vector can autonomously replicate in eukaryotic cells and is therefore maintained in transfected cells upon G418 selection without the need for being integrated into the host cell genome. The cDNA coding for TRK-T1 was obtained from phage 24 (Greco, A., et al. (1992). *Oncogene* 7, 237-242.) which had been shown to contain a cDNA insert capable of foci formation upon transfection into NIH3T3 cells. The cDNA insert was excised from phage 24 DNA by digestion with EcoRI. pCEP9B was linearized by digestion with HindIII, which cuts within the polylinker of the plasmid. The ends of the insert fragment and the linearized vector were blunted with the Klenow fragment of DNA polymerase I and subsequently ligated using T4 ligase. Recombinant plasmids resulting from this ligation reaction were analyzed by restriction analysis. Taking advantage of two asymmetric HindIII sites present in the TRK-T1 cDNA and a BamHI site localized in the vector (see Fig. 1), the orientation of the TRK-T1 cDNA insert was determined by HindIII/BamHI digestion. Plasmids containing the TRK-T1 cDNA in the sense and antisense orientation relative to the CMV promotor were isolated and designated pC24A (see Fig. 1) and pC24B (not shown), respectively.

### Example B:

### PC12 Cell Cultures

The rat PC12 pheochromocytoma cell line was obtained from ATCC. The cells were maintained in RPMI-160 medium supplemented with 5% fetal calf serum, 10% horse serum, penicillin and streptomycin (100 U.I./ml) at 37°C in a humidified atmosphere containing 5% CO₂. For treatment with NGF, the cells were plated onto collagen-coated plastic dishes. NGF was supplied to the cells at a concentration of 50 ng/ml. Transfected cells were seeded onto plastic dishes.

### Example C:

### Introduction of Expression Vectors pC24A and pC24B Containing the TRK-T1 Oncogene into PC12 Cells

Exponentially growing PC12 cells were resuspended in RPMI-160 medium containing 50% fetal calf serum at a density of 1.5-3x10⁷ cells/ml, mixed with 50 µg of vector DNA and incubated at 4°C for 15 min. Electroporation was carried out with a Bio-Rad Gene Pulser (Bio-Rad Laboratories, Richmond, CA), with the settings of 960 µF, 250 Volts and applying 3 pulses. The vector DNA used for electroporation was derived from plasmids pC24A and pC24B (see Example A), carrying the TRK-T1 cDNA in sense (pC24A) or in antisense (pC24B) orientation with respect to the promotor. After electroporation, the cells were incubated on ice for 20 min., diluted in PC12 conditioned medium and seeded on plastic Petri dishes. The antibiotic G418 was added either immediatly after plating or 24-48 hours later at a concentration of 400 µg/ml. The selective medium was renewed weekly.

### Example D:

### Introduction of other Expression Vectors Carrying the TRK-T1 and TRK Oncogenes into PC12 Cells

DNA derived from plasmids p24EA or p24EB (Greco A., et al. (1992). *Oncogene* 7, 237-242.), containing the *TRK*-T1 oncogene in sense (p24EA) or antisense (p24EB) orientation, respectively, or DNA derived from vector pDM16 (Martin-Zanca, D., et al. (1986). *Nature* 319, 743-748) containing the *TRK* gene, was mixed with plasmid DNA derived from pSV2neo (Southern, P. J., Berg, P. (1982). *J. Mol. Appl. Genet.* 1, 327-341.) at a ratio of 5:1. Subsequently, the mixture was cotransfected into PC12 cells by electroporation. The total amount of plasmid DNA was 50 µg. The conditions for electroporation and the treatment of the cells after introduction of the vector DNA were as described in Example C.

### Example E:

### Phenotype of Transformed PC12 Cell Cultures

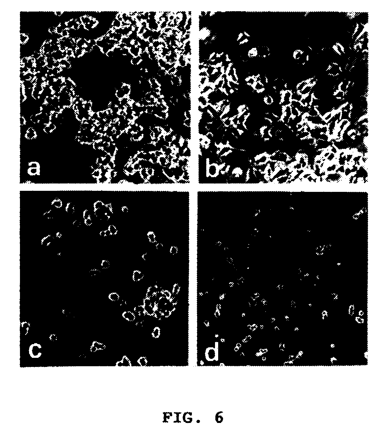

Three days after transfection of PC12 cells with the vectors p24EA (not shown) or vector pC24A, containing the *TRK*-T1 oncogene in sense orientation or with vector pDM16, containing the *TRK* oncogene in sense orientation, differentiated cells extending long neurites could be observed in culture (Fig. 2 A, 9 A). These cells were indistinguishable in their morphology from untransfected PC12 cells that had been treated with NGF (Fig. 2 B). It was found that the two TRK-T1-encoding vectors differ in their efficiency to promote neuronal differentiation. While transfection of vector pC24A produced 312 differentiated cells/µg of DNA, the number of differentiated cells was about ten fold lower when using the same amount of vector p24EA for transfection. Cultures of cells transfected with the vectors p24EB (not shown) and pC24B (Fig. 2 C), containing the *TRK*-T1 oncogene in antisense orientation, displayed a morphology similar to untransfected normal PC12 cell cultures (Fig. 2 D). Upon prolonged cultivation, the cultures that were differentiated by the NTRK1-derived oncogenes displayed cellular degeneration. The morphology of PC12 cells transfected with vector pC24A after 10 days in culture is shown in Fig. 5. The cells underwent degenerative processes, characterized by an increase in size, the appearance of vacuoles and detachment from the plate. This behaviour was consistently observed in different experiments performed under identical conditions (Fig. 5 A, B). This is in contrast to NGF-treated untransfected PC12 cell cultures, which do not display degenerative processes. Rather, the cells remain stably differentiated and viable as long as NGF is provided. Moreover, the cells loose their neurites and regain their proliferative capabilities upon removal of NGF (Greene, L. A., and Tischler, A. S. (1976). *Proc. Natl. Acad. Sci. USA* 73, 2424.)

### Example F:

### Detection of TRK-T1 cDNA in Transfected Cells

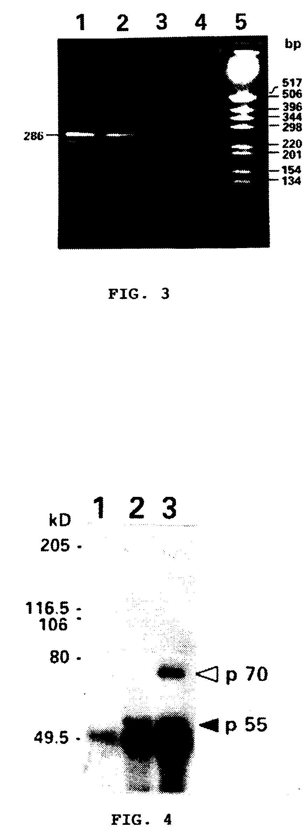

The presence of the *TRK*-T1 oncogene in PC12 cells transfected with the vector pC24A was verified using the polymerase chain reaction. Low molecular weight DNA was extracted 3-5 days after transfection from transfected cells as described (Hirt, B. (1967). J. *Mol. Biol.* 26, 365-369.). An aliquot of the isolated DNA was subjected to PCR amplification using the primers PR1 (5'-GCTGAGAAAAGAGACTTAAT-3') and PR2 (5'-CATCACTGAAGTATTGTG-3'), that encompass a 286 bp fragment including the TPR/NTRK1 junction of the TRK-T1 cDNA (Greco, A., et al. (1992). *Oncogene* 7, 237-242.). Amplification was carried out in a volume of 100 pl in 1 x PCR buffer (Perkin Elmer) supplemented with dNTPs, each at a concentration of 2.5 mM, primers PR1 and PR2, each at a concentration of 45 pM, 2 nM MgCl₂ and 2.5 units of Taq polymerase (Perkin Elmer). After a denaturation step at 95°C for 30 sec., 30 cycles of amplification were carried out (95°C for 1 min., 55°C for 30 sec., 72°C for 1.5 min.), followed by an extention reaction at 72°C. Fifteen microliters of the samples were subjected to gel electrophoresis on a 4% Nusieve agarose 3:1 gel. Analysis of the reaction products by ethidium bromide staining of the gel revealed the amplification of a 286 bp DNA fragment from material isolated from pC24A-transfected PC12 cells (Fig. 3, lanes 1, 2). No amplification products could be detected performing PCR on material derived from cells transfected with a control plasmid (pLM6; Fig. 2, lane 3), or from mock transfectants (Fig. 2, lane 4).

### Example G:

### Detection of TRK-T1 and TRK Protein in Transfected Cells

The presence of the oncogene products in PC12 cells transfected with the vectors pC24A and pDM16 was monitored by Western blotting and detection of the oncoproteins with an antiserum specifically reacting with the 14 carboxy-terminal amino acid residues of the NTRK1 protein (Greco, A., et al. (1992). *Oncogene* 7, 237-242.). Preparation of the material from transfected cells used for Western blotting and the detection procedure were carried out as described (Bongarzone, I., Pierotti, M. A., Monzini, N., Mondellini, P., Manenti, G., Donghi, R., Pilotti, S., Grieco, M., Santoro, M., Fusco, A., Vecchio, G., and Della Porta, G. (1989). *Oncogene* 4, 1457-1462.). Analysis of the Western blot revealed expression of the 55 kDa TRK-T1 protein (Fig 4, lane 1) and the 70 kDa TRK protein (Fig. 4, lane 2) in cells transfected with the vectors pC24A and pDM16, respectively.

### Example H:

### Inhibition of Neuronal Differentiation with K252a

To compare the NGF-induced neuronal differentiation with the events induced by transformation of neuronal cells with *NTRK1*-derived oncoproteins on the biochemical level, PC12 cells that were either treated with NGF or had been transfected with the vectors pC24A and pDM16 were cultured in the absence or presence of the alkaloid-like kinase inhibitor K252a, which specifically interferes with the phosphorylation of the NTRK1 protein and inhibits NGF action on PC12 cells (Hashimoto, S. (1988). *J. Cell* *Biol.* 107, 1531-1539.). PC12 cells cultivated with NGF (50 ng/ml; Fig. 6 A, B) for 4 days revealed normal differentiation in the absence of the inhibitor K252a (Fig. 6 B). However, differentiation was completely blocked by the addition of K252a (200 nM) to the medium (Fig. 6 A). Similarly, PC12 cells transfected with vector pC24A and cultivated for 4 days (Fig. 6 C, D) displayed differentiation to a neuronal phenotype in the absence of K252a (Fig. 6D), but were lacking any characteristics of neuronal differentiation when grown in the presence of K252a (Fig. 6 C). The same effect was also found for PC12 cells that had been transfected with vector pDM16 and grown for 3 days (Fig. 9 A, B). K252a addition completely abolished the differentiation of the transformants (Fig. 9 B), whereas in the absence of the inhibitor the cells were found to bear long neuritic processes and a neuronal morphology (Fig. 9A).

### Example I:

### Detection of the VGF8 Gene Product in Transformed Cells

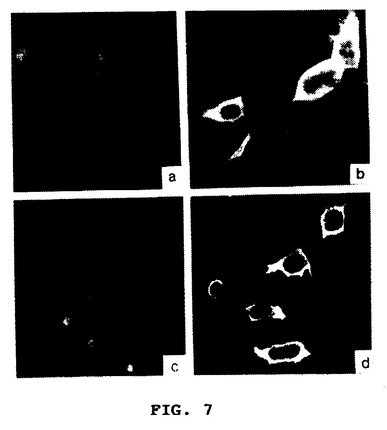

VGF8 expression as a result of the induction of neuronal differentiation was monitored using immunocytochemistry. PC12 cells that had been transformed with the *TRK*-T1 oncogene and were subsequently grown in slide chambers (Gibco BRL) for three days (Fig. 7 A, B), or untransfected PC12 cells grown in slide chambers in the presence of NGF (50 ng/ml) for 1 day (Fig. 7 C, D), were fixed with acetone, incubated with the antiserum aVX which is specific for the VGF8 gene product (Possenti, R., et al. (1989). *EMBO J.* 8, 2217-2223.) or with preimmune serum, repeatedly washed and further incubated with flourescein-labelled goat anti-rabbit antibodies (Meloy, Springfield, VA). TRK-T1-transformed PC12 cells as well as NGF-treated PC12 cells revealed strong immunoreactivity with the aVX antiserum, indicating a high level expression of the VGF8 protein Fig. 7 B, D). No staining could be observed with both cell types when the preimmune serum was used as first antibody (Fig. 7 A, C).

### Example J:

### Treatment of Transformed Cells with Antibodies to NGF

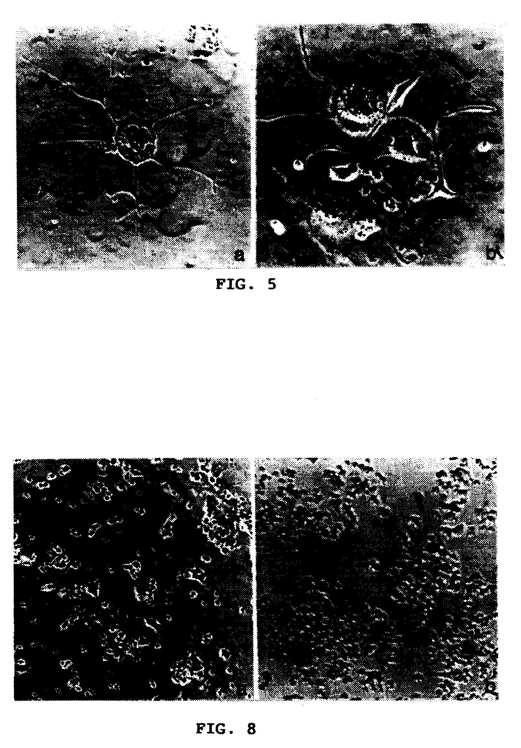

In order to investigate, whether transfection of neuronal cells might induce the production of NGF by the transformants, thereby creating an autocrine circuit that would be responsible for the observed neuronal differentiation, PC12 cells that had been converted to the neuronal phenotype by transformation with the TRK-T1 gene or by NGF treatment, were cultured for 3 days in the presence of the antibody D11 (Cattaneo, A., et al. (1988). *J. Neurochem.* 50, 1003-1010; Fig. 8 A, B), which is specific for NGF and blocks its biological activity. Despite the presence of the D11 antibodies, the *TRK*-T1-transformed cells continued to display a neuronal morphology (Fig. 8 A), whereas cultures treated with NGF were converted to an undifferentiated state (Fig. 8 B). In accord with these observations, no induction of neuronal differentiation could be detected with untransfected PC12 cells that were grown in conditioned medium derived from pC24A-transfected cells (not shown).

## Claims

1. Neuronal cells characterized in that they comprise a gene coding for an oncogenic derivative of the NGF-receptor NTRK1, said gene causing neuronal differentiation of said cells in the absence of NGF.

2. Cells according to claim 1 that are PC12 pheochromocytoma cells.

3. Cells according to claim 1 and 2 characterized in that they comprise a gene coding for the NTRK1-related oncoprotein TRK-T1.

4. Cells according to claim 1 and 2 characterized in that they comprise the original *TRK* oncogene.

5. A method of obtaining neuronal cells displaying NGF-independent neuronal differentiation characterized by transfection of vectors containing a gene coding for an oncogenic derivative of the NGF-receptor NTRK1 into neuronal host cells.

6. A method according to claim 5, wherein the neuronal cells are PC12 pheochromocytoma cells.

7. A method for screening compounds having anti-tyrosine kinase activity comprising the culture of the cells of claims 1-4 in the presence of the compound to be tested and the analysis of the cultured cells for the absence of neuronal differentiation.

## Patentansprüche

1. Neuronale Zellen, dadurch gekennzeichnet, daß sie ein Gen umfassen, das ein onkogenes Derivat des NGF-Rezeptors NTRK1 codiert, wobei das Gen die neuronale Differenzierung dieser Zellen in Abwesenheit von NGF verursacht.

2. Zellen gemäß Anspruch 1, die PC12-Pheochromocytomazellen sind.

3. Zellen nach Anspruch 1 und 2, dadurch gekennzeichnet, daß sie ein Gen umfassen, das ein NTRK1-verwandtes Onkoprotein TRK-T1 codiert.

4. Zellen nach Anspruch 1 und 2, dadurch gekennzeichnet, daß sie das ursprüngliche TRK-Onkogen umfassen.

5. Ein Verfahren zum Gewinnen neuronaler Zellen, die NGF-unabhängige neuronale Differenzierung zeigen, gekennzeichnet durch die Transfektion von Vektoren in neuronale Wirtszellen, wobei die Vektoren ein Gen enthalten, das ein onkogenes Derivat des NGF-Rezeptors NTRK1 codiert.

6. Verfahren nach Anspruch 5, wobei die neuronalen Zellen PC12-Pheochromocytomazellen sind.

7. Verfahren zum Screening von Verbindungen mit Antityrosinkinaseaktivität, umfassend die Kultur der Zellen gemäß Ansprüchen 1 bis 4 in Gegenwart der zu testenden Verbindung und die Analyse der kultivierten Zellen auf die Abwesenheit neuronaler Differenzierung.

## Revendications

1. Cellules neuronales, caractérisées en ce qu'elles comprennent un gène codant pour un dérivé oncogénique de NTRK1 du récepteur de NGF, ledit gène entraînant la différenciation neuronale desdites cellules en l'absence de NGF.

2. Cellules selon la revendication 1, caractérisées en ce qu'il s'agit de cellules de phéochromocytome PC12.

3. Cellules selon les revendications 1 et 2, caractérisées en ce qu'elles comprennent un gène codant pour l'oncoprotéine TRK-T1 apparentée à NTRK1.

4. Cellules selon les revendications 1 et 2, caractérisées en ce qu'elles comprennent l'oncogène *TRK* d'origine.

5. Méthode d'obtention de cellules neuronales présentant une différenciation neuronale indépendante de NGF, caractérisée par la transfection de vecteurs contenant un gène codant pour un dérivé oncogénique de NTRK1 du récepteur de NGF dans des cellules neuronales hôtes.

6. Méthode selon la revendication 5, caractérisée en ce que les cellules neuronales sont des cellules de phéochromocytome PC12.

7. Méthode de criblage de composés ayant une activité anti-tyrosine kinase, comprenant la culture des cellules des revendications 1-4 en présence du composé à tester et l'analyse des cellules cultivées pour l'absence de différenciation neuronale.
